(19) Europäisches Patentamt
European Patent Office
Office européen des brevets

(11) EP 1 177 798 A2

(12) EUROPEAN PATENT APPLICATION

(43) Date of publication:
**06.02.2002 Bulletin 2002/06**

(51) Int Cl.⁷: **A61K 45/06**

(21) Application number: **01306455.5**

(22) Date of filing: **27.07.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.07.2000 US 221718 P**

(71) Applicant: **Pfizer Products Inc.**
**Groton, Connecticut 06340 (US)**

(72) Inventors:
- **Watsky, Eric Jacob**
  **Groton, Connecticut 06340 (US)**
- **Coe, Jotham Wadsworth**
  **Groton, Connecticut 06340 (US)**
- **Harrigan, Edmund Patrick**
  **Groton, Connecticut 06340 (US)**
- **O'Neill, Brian Thomas**
  **Groton, Connecticut 06340 (US)**
- **Sands, Steven Bradley**
  **Groton, Connecticut 06340 (US)**

(74) Representative:
**Simpson, Alison Elizabeth Fraser et al**
**Urquhart-Dykes & Lord, 30 Welbeck Street**
**London W1G 8ER (GB)**

(54) **A pharmaceutical composition for the treatment of attention deficit hyperactivity disorder(ADHD) comprising a nicotine receptor partial agonist and anti-ADHD agent**

(57) Pharmaceutical compositions are disclosed for the treatment of attention deficit hyperactivity disorder (ADHD). The pharmaceutical compositions are comprised of a therapeutically effective combination of a nicotine receptor partial agonist and an anti-ADHD agent and a pharmaceutically acceptable carrier. The method of using these compounds is also disclosed.

EP 1 177 798 A2

## Description

Background of the Invention

The present invention relates to pharmaceutical compositions for the treatment of Attention Deficit Hyperactivity Disorder (ADHD) in a mammal (e.g. human) comprising a nicotine receptor partial agonist (NRPA) in combination with one of the following: an alpha-1($\alpha$1) adrenergic receptor ligand, an alpha-2($\alpha$2) adrenergic receptor ligand, a $D_2$ receptor agonist, a $5HT_{1A}$ receptor agonist, a cholinesterase inhibitor, or a norepinephrine re-uptake inhibitor (NERUI). The term NRPA refers to all chemical compounds which bind at neuronal nicotinic acetylcholine specific receptor sites in mammalian tissue and elicit a partial agonist response. A partial agonist response is defined here to mean a partial, or incomplete functional effect in a given functional assay. Additionally, a partial agonist will also exhibit some degree of antagonist activity by its ability to block the action of a full agonist (Feldman, R.S., Meyer, J.S. & Quenzer, L.F. Principles of Neuropsychopharmacology, 1997; Sinauer Assoc. Inc.). The present invention may be used to treat mammals (e.g. humans) for ADHD.

Evidence in the literature suggests that nicotine may improve attentiveness (for review, see Levin, E., Psychopharmacology 108:417-431, 1992). In animal studies, nicotine can reverse deficits in working memory in brain-lesioned rats (Levin et al., Cognitive Brain Research 1:137-143, 1993) and also improves performance on serial choice tasks which are thought to partially model symptoms of ADHD (Muir, et al., Psychopharmacology 118;82-92, 1995). In humans, nicotine significantly improves clinical ADHD symptoms as measured by the Clinical Global Impression scale (Levin, et al., Psychopharmacology 123:55-63, 1996). Thus nicotinic agents may have therapeutic utility in the treatment of ADHD.

Additionally, symptoms associated with ADHD have been shown to be relieved by a wide variety of other agents, including but not limited to catecholamine releasing drugs such as methylphenidate (Ritalin), by postsynaptic $\alpha$2 adrenergic receptor agonists (e.g. clonidine), by $\alpha$1 adrenergic receptor agonists (e.g. modafinil (Provigil)), by $5HT_{1A}$ receptor agonists (e.g. sunipetron, and bis-azabicyclic compounds of the Formula I disclosed in United States Patent 5,122,525,) as defined below and incorporated herein and NERUIs (e.g. tomoxetine) and presynaptic $\alpha$2 adrenergic receptor antagonists which increase norepinephrine (NE) release.

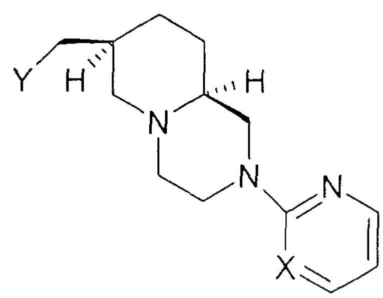

I

Summary of Invention

The present invention relates to a pharmaceutical composition for the treatment of Attention Deficit Hyperactivity Disorder comprising

(a) a nicotine receptor partial agonist or a pharmaceutically acceptable salt thereof;
(b) an $\alpha_2$ adrenergic receptor ligand, an $\alpha_1$ adrenergic receptor ligand, a $D_2$ receptor agonist, a NEURI, a $5HT_{1A}$ receptor agonist, and/or a cholinesterase inhibitor or a pharmaceutically acceptable salt thereof; and
(c) a pharmaceutically acceptable carrier;

wherein the active agents "a" and "b" above are present in amounts that render the composition effective in treating Attention Deficit Hyperactivity Disorder (ADHD).

Examples of $D_2$ receptor agonists that can be used in the pharmaceutical compositions of this invention include, but are not limited to, pergolide (Permax), bromocriptine (Parlodel), ropinirole (Requip), and pramipexole (Mirapex), pemoline (Cylert), cabergoline (Dostinex), amphetamine (Adderall), dextroamphetamine (Dexedrine) and methylphenidate (Ritalin) and their pharmaceutically acceptable salts.

Examples of $5HT_{1A}$ receptor agonists that can be used in the pharmaceutical compositions of this invention include, but are not limited to: (a) sunepitron, and other bis-azabicyclic compounds disclosed in U.S. patent 5,122,525 and their pharmaceutically acceptable salts, (b) buspirone (Buspar) (c) gepirone; (c) ipsapirone; and (d) and flesinoxan.

Examples of $\alpha_2$ adrenergic receptor antagonists that can be used in the compositions of this invention include, but are not limited to, yohimbine, idazoxan and clonidine (Catapres) and their pharmaceutically acceptable salts.

Examples of $\alpha_1$ adrenergic receptor agonists that can be used in the compositions of this invention include, but are not limited to, modafinil (Provigil) and their pharmaceutically acceptable salts.

Examples of norepinephrine re-uptake inhibitors (NEURI) that can be used in the compositions of this invention include, but are not limited to tomoxetine, bupropion (Wellbutrin), and venlafaxine (Effexor) and their pharmaceutically acceptable salts.

Examples of cholinesterase inhibitors that can be used in the compositions of this invention include, but are not limited to donepezil (Aricept), tacrine (Cognex™), rivastigmine (Exelon™), physostigmine (Synapton), galanthamine (Reminyl), metrifonate (Promem), neostigmine (Prostigmin), and icopezil and their pharmaceutically acceptable salts.

In another more specific embodiment of this invention, the nicotine receptor partial agonist is selected from:

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1,5]diazocin-8-one;
9-(2-propyl)- 1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1 ,5-methano-pyrido[1,2a][1,5]diazocin-8-one;
9-(3,5-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(2,5-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.$0^{2,10}$.$0^{4,8}$]pentadeca-2(10),3,8-triene;
5-oxo-6,13-diazatetracyclo[9.3.1.$0^{2,10}$.$0^{4,8}$]pentadeca-2(10),3,8-triene;
6-oxo-5,7,13-triazatetracyclo[9.3.1.$0^{2,10}$.$0^{4,8}$]pentadeca-2(10),3,8-triene;
4,5-difluoro-10-aza-tricyclo[6.3.1.$0^{2,7}$]dodeca-2(7),3,5-triene;
5-fluoro-10-aza-tricyclo[6.3.1.$0^{2,7}$]dodeca-2(7),3,5-triene-4-carbonitrile;
4-ethynyl-5-fluoro-10-aza-tricyclo[6.3.1.$0^{2,7}$]dodeca-2(7),3,5-triene;
5-ethynyl-10-aza-tricyclo[6.3.1.$0^{2,7}$]dodeca-2(7),3,5-triene-4-carbonitrile;
6-methyl-5-thia-5-dioxa-6,13-diazatetracyclo[9.3.1.$0^{2,10}$.$0^{4,8}$]pentadeca-2(10),3,8-triene;
10-aza-tricyclo[6.3.1.$0^{2,7}$]dodeca-2(7),3,5-triene;
4-fluoro-10-aza-tricyclo[6.3.1.$0^{2,7}$]dodeca-2(7),3,5-triene;
4-methyl-10-aza-tricyclo[6.3.1.$0^{2,7}$]dodeca-2(7),3,5-triene;
4-trifluoromethyl-10-aza-tricyclo[6.3.1.$0^{2,7}$]dodeca-2(7),3,5-triene;
4-nitro-10-azatricyclo[6.3.1.$0^{2,7}$]dodeca-2(7),3,5-triene;
7-methyl-5,7,13-triazatetracyclo[9.3.1.$0^{2,10}$.$0^{4,8}$]pentadeca-2(10),3,5,8-tetraene;
6-methyl-5,7,13-triazatetracyclo[9.3.1.$0^{2,10}$.$0^{4,8}$]pentadeca-2(10),3,5,8-tetraene;
6,7-dimethyl-5,7,13-triazatetracyclo[9.3.1.$0^{2,10}$.$0^{4,8}$]pentadeca-2(10),3,5,8-tetraene;

6-methyl-7-phenyl-5,7,13-triazatetracyclo[9.3.1.$0^{2,10}$.$0^{4,8}$]pentadeca-2(10)3,5,8-tetraene;
6,7-dimethyl-5,8,14-triazatetracyclo[10.3.1.$0^{2,11}$.$0^{4,9}$]hexadeca-2(11),3,5,7,9-pentaene;
5,8,14-triazatetracyclo[10.3.1.$0^{2,11}$.$0^{4,9}$]hexadeca-2(11),3,5,7,9-pentaene;
14-methyl-5,8,14-triazatetracyclo[10.3.1.$0^{2,11}$.$0^{4,9}$]hexadeca-2(11),3,5,7,9-pentaene;
5-oxa-7,13-diazatetracyclo[9.3.1.$0^{2,10}$.$0^{4,8}$]pentadeca-2(10),3,6,8-tetraene;
6-methyl-5-oxa-7,13-diazatetracyclo[9.3.1.$0^{2,10}$.$0^{4,8}$]pentadeca-2(10),3,6,8-tetraene;
4-chloro-10-azatricyclo[6.3.1.$0^{2,7}$]dodeca-2(7),3,5-triene;
10-azatricyclo[6.3.1.$0^{2,7}$]dodeca-2(7),3,5-trien-4-yl cyanide;
1-(10-azatricyclo[6.3.1.$0^{2,7}$]dodeca-2(7),3,5-trien-4-yl)-1-ethanone;
10-azatricyclo[6.3.1.$0^{2,7}$]dodeca-2(7),3,5-trien-4-ol;
7-methyl-5-oxa-6,13-diazatetracyclo[9.3.1.$0^{2,10}$.$0^{4,8}$]pentadeca-2,4(8),6,9-tetraene;
4,5-dichloro-10-azatricyclo[6.3.1.$0^{2,7}$]dodeca-2(7),3,5-triene;
11-azatricyclo[7.3.1.$0^{2,7}$]trideca-2(7),3,5-triene-5-carbonitrile;
1-[11-azatricyclo[7.3,1.$0^{2,7}$]trideca-2(7),3,5-trien-5-yl]-1-ethanone;
1-[11-azatricyclo[7.3.1.$0^{2,7}$]trideca-2(7),3,5-trien-5-yl]-1-propanone;
4-fluoro-11-azatricyclo[7.3.1.$0^{2,7}$]trideca-2(7),3,5-triene-5-carbonitrile;
5-fluoro-11-azatricyclo[7.3.1.$0^{2,7}$]trideca-2(7),3,5-triene-4-carbonitrile;
6-methyl-7-thia-5,14-diazatetracyclo[10.3.1.$0^{2,10}$.$0^{4,8}$]hexadeca-2(10),3,5,8-tetraene;
6-methyl-5,7,14-triazatetracyclo[10.3.1.$0^{2,10}$.$0^{4,8}$]hexadeca-2(10),3,5,8-tetraene;
6,7-dimethyl-5,7,14-triazatetracyclo[10.3.1.$0^{2,10}$.$0^{4,8}$]hexadeca-2(10),3,5,8-tetraene;
5,7,14-triazatetracyclo[10.3.1.$0^{2,10}$.$0^{4,8}$]hexadeca-2(10),3,5,8-tetraene;
5,6-dimethyl-5,7,14-triazatetracyclo[10.3.1.$0^{2,10}$.$0^{4,8}$]hexadeca-2(10),3,6,8-tetraene;
5-methyl-5,7,14-triazatetracyclo[10.3.1.$0^{2,10}$.$0^{4,8}$]hexadeca-2(10),3,6,8-tetraene;
6-(trifluoromethyl)-7-thia-5,14-diazatetracyclo[10.3.1.$0^{2,10}$.$0^{4,8}$]hexadeca-2(10),3,5,8-tetraene;
5,8,15-triazatetracyclo[11.3.1.$0^{2,11}$.$0^{4,9}$]heptadeca-2(11),3,5,7,9-pentaene;
7-methyl-5,8,15-triazatetracyclo[11.3.1.$0^{2,11}$.$0^{4,9}$]heptadeca-2(11),3,5,7,9-pentaene;
6-methyl-5,8,15-triazatetracyclo[11.3.1.$0^{2,11}$.$0^{4,9}$]heptadeca-2(11),3,5,7,9-pentaene;
6,7-dimethyl-5,8,15-triazatetracyclo[11.3.1.$0^{2,11}$.$0^{4,9}$]heptadeca-2(11),3,5,7,9-pentaene;
7-oxa-5,14-diazatetracyclo[10.3.1.$0^{2,10}$.$0^{4,8}$]hexadeca-2(10),3,5,8-tetraene;
6-methyl-7-oxa-5,14-diazatetracyclo[10.3.1.$0^{2,10}$.$0^{4,8}$]hexadeca-2(10),3,5,8-tetraene;
5-methyl-7-oxa-6,14-diazatetracyclo[10.3.1.$0^{2,10}$.$0^{4,8}$]hexadeca-2(10),3,5,8-tetraene;
6-methyl-5-oxa-7,14-diazatetracyclo[10.3.1.$0^{2,10}$.$0^{4,8}$]hexadeca-2(10),3,6,8-tetraene;
7-methyl-5-oxa-6,14-diazatetracyclo[10.3.1.$0^{2,10}$.$0^{4,8}$]hexadeca-2(10),3,6,8-tetraene;
4,5-difluoro-11-azatricyclo[7.3.1.$0^{2,7}$]trideca-2(7),3,5-triene;
4-chloro-5-fluoro-11-azatricyclo[7.3.1.$0^{2,7}$]trideca-2(7),3,5-triene;
5-chloro-4-fluoro-11-azatricyclo[7.3.1.$0^{2,7}$]trideca-2(7),3,5-triene;
4-(1-ethynyl)-5-fluoro-11-azatricyclo[7.3.1.$0^{2,7}$]trideca-2(7),3,5-triene;
5-(1-ethynyl)-4-fluoro-11-azatricyclo[7.3.1.$0^{2,7}$]trideca-2(7),3,5-triene;
5,6-difluoro-11-aza-tricyclo[7.3.1.$0^{2,7}$]trideca-2,4,6-triene;
6-trifluoromethyl-11-aza-tricyclo[7.3.1.$0^{2,7}$]trideca-2,4,6-triene;
6-methoxy-11-aza-tricyclo[7.3.1.$0^{2,7}$]trideca-2(7),3,5-triene;
11-aza-tricyclo[7.3.1.$0^{2,7}$]trideca-2(7),3,5-trien-6-ol;
6-fluoro-11-aza-tricyclo[7.3.1.$0^{2,7}$]trideca-2(7),3,5-triene;
11-aza-tricyclo[7.3.1.$0^{2,7}$]trideca-2(7),3,5-trien-5-ol;
4-nitro-11-aza-tricyclo[7.3.1.$0^{2,7}$]trideca-2(7),3,5-triene;
5-nitro-11-aza-tricyclo[7.3.1.$0^{2,7}$]trideca-2(7),3,5-triene;
5-fluoro-11-aza-tricyclo[7.3.1.$0^{2,7}$]trideca-2(7),3,5-triene; and
6-hydroxy-5-methoxy-11-aza-tricyclo[7.3.1.$0^{2,7}$]trideca-2(7),3,5-triene and their pharmaceutically acceptable salts and their optical isomers.

Preferably, the nicotine receptor partial agonist is selected from

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,23,4,5,6-hexahydro-1,5-methano-pyrido[12-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
6-methyl-5-thia-5-dioxa-6,13-diazatetracyclo[9.3.1.$0^{2,10}$.$0^{4,8}$]pentadeca-2(10),3,8-triene;
4-fluoro-10-aza-tricyclo[6.3.1.$0^{2,7}$]dodeca-2(7),3,5-triene;
4-trifluoromethyl-10-aza-tricyclo[6.3.1.$0^{2,7}$]dodeca-2(7),3,5-triene;
4-nitro-10-azatricyclo[6.3.1.$0^{2,7}$]dodeca-2(7),3,5-triene;
6-methyl-5,7,13-triazatetracyclo[9.3.1.$0^{2,10}$.$0^{4,8}$]pentadeca-2(10),3,5,8-tetraene;
6,7-dimethyl-5,8,14-triazatetracyclo[10.3.1.$0^{2,11}$.$0^{4,9}$]hexadeca-2(11),3,5,7,9-pentaene;
5,8,14-triazatetracyclo[10.3.1.$0^{2,11}$.$0^{4,9}$]hexadeca-2(11),3,5,7,9-pentaene;
5-oxa-7,13-diazatetracyclo[9.3.1.$0^{2,10}$.$0^{4,8}$]pentadeca-2(10),3,6,8-tetraene;
6-methyl-5-oxa-7,13-diazatetracyclo[9.3.1.$0^{2,10}$.$0^{4,8}$]pentadeca-2(10),3,6,8-tetraene;
10-azatricyclo[6.3.1.$0^{2,7}$]dodeca-2(7),3,5-trien-4-yl cyanide;
1-(10-azatricyclo[6.3.1.$0^{2,7}$]dodeca-2(7),3,5-trien-4-yl)-1-ethanone;
11-azatricyclo[7.3.1.$0^{2,7}$]trideca-2(7),3,5-triene-5-carbonitrile;
1-[11-azatricyclo[7.3.1.$0^{2,7}$]trideca-2(7),3,5-trien-5-yl]-1-ethanone;
1-[11-azatricyclo[7.3.1.$0^{2,7}$]trideca-2(7),3,5-trien-5-yl]-1-propanone;
4-fluoro-11-azatricyclo[7.3.1.$0^{2,7}$]trideca-2(7),3,5-triene-5-carbonitrile;
5-fluoro-11-azatricyclo[7.3.1.$0^{2,7}$]trideca-2(7)3,5-triene-4-carbonitrile;
6-methyl-7-thia-5,14-diazatetracyclo[10.3.1.$0^{2,10}$.$0^{4,8}$]hexadeca-2(10),3,5,8-tetraene;
6-methyl-5,7,14-triazatetracyclo[10.3.1.$0^{2,10}$.$0^{4,8}$]hexadeca-2(10),3,5,8-tetraene;
6,7-dimethyl-5,7,14-triazatetracyclo[10.3.1.$0^{2,10}$.$0^{4,8}$]hexadeca-2(10),3,5,8-tetraene;
6-methyl-7-oxa-5,14-diazatetracyclo[10.3.1.$0^{2,10}$.$0^{4,8}$]hexadeca-2(10),3,5,8-tetraene;
6-methyl-5-oxa-7,14-diazatetracyclo[10.3.1.$0^{2,10}$.$0^{4,8}$]hexadeca-2(10),3,6,8-tetraene;
5,6-difluoro-11-aza-tricyclo[7.3.1.$0^{2,7}$]trideca-2,4,6-triene;
6-trifluoromethyl-11-aza-tricyclo[7.3.1.$0^{2,7}$]trideca-2,4,6-triene;
6-methoxy-11-aza-tricyclo[7.3.1.$0^{2,7}$]trideca-2(7),3,5-triene;
6-fluoro-11-aza-tricyclo[7.3.1.$0^{2,7}$]trideca-2(7),3,5-triene; and
11-aza-tricyclo[7.3.1.$0^{2,7}$]trideca-2(7),3,5-trien-5-ol and their pharmaceutically acceptable salts and their optical isomers.

The present invention also relates to a method of treating ADHD in a mammal comprising administering to said mammal respectively an anti-ADHD attenuating effective amount of a pharmaceutical composition comprising

(a) a nicotine receptor partial agonist or a pharmaceutically acceptable salt thereof; and
(b) an $\alpha_2$ adrenergic receptor ligand, an $\alpha_1$ adrenergic receptor ligand, a $D_2$ receptor agonist, a NEURI, a $5HT_{1A}$ receptor agonist, and/or a cholinesterase inhibitor or a pharmaceutically acceptable salt thereof;

wherein the active ingredients (a) and (b) are present in amounts that render the composition effective in the treatment of ADHD.

In another more specific embodiment of this invention the nicotine receptor partial agonist is selected from:

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,56-hexahydro-1,5-methano-pyrido[12a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one;

9-(2-propenyl)-1,2,3,4,5,6-hexahydro-1,5-methano-pyrido[1,2a][1,5]diazocin-8-one;
9-(2-propyl)- 1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(26-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one;
9-(3,5-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(25-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.$0^{2,10}$.$0^{4,8}$]pentadeca-2(10),3,8-triene;
5-oxo-6,13-diazatetracyclo[9.3.1.$0^{2,10}$.$0^{4,8}$]pentadeca-2(10),3,8-triene;
6-oxo-5,7,13-triazatetracyclo[9.3.1.$0^{2,10}$.$0^{4,8}$]pentadeca-2(10),3,8-triene;
4,5-difluoro-10-aza-tricyclo[6.3.1.$0^{2,7}$]dodeca-2(7),3,5-triene;
5-fluoro-10-aza-tricyclo[6.3.1.$0^{2,7}$]dodeca-2(7),3,5-triene-4-carbonitrile;
4-ethynyl-5-fluoro-10-aza-tricyclo[6.3.1.$0^{2,7}$]dodeca-2(7),3,5-triene;
5-ethynyl-10-aza-tricyclo[6.3.1.$0^{2,7}$]dodeca-2(7),3,5-triene-4-carbonitrile;
6-methyl-5-thia-5-dioxa-6,13-diazatetracyclo[9.3.1.$0^{2,10}$.$0^{4,8}$]pentadeca-2(10),3,8-triene;
10-aza-tricyclo[6.3.1.$0^{2,7}$]dodeca-2(7),3,5-triene;
4-fluoro-10-aza-tricyclo[6.3.1.$0^{2,7}$]dodeca-2(7),3,5-triene;
4-methyl-10-aza-tricyclo[6.3.1.$0^{2,7}$]dodeca-2(7),3,5-triene;
4-trifluoromethyl-10-aza-tricyclo[6.3.1.$0^{2,7}$]dodeca-2(7),3,5-triene;
4-nitro-10-azatricyclo[6.3.1.$0^{2,7}$]dodeca-2(7),3,5-triene;
7-methyl-5,7,13-triazatetracyclo[9.3.1.$0^{2,10}$.$0^{4,8}$]pentadeca-2(10),3,5,8-tetraene;
6-methyl-5,7,13-triazatetracyclo[9.3.1.$0^{2,10}$.$0^{4,8}$]pentadeca-2(10),3,5,8-tetraene;
6,7-dimethyl-5,7,13-triazatetracyclo[9.3.1.$0^{2,10}$.$0^{4,8}$]pentadeca-2(10),3,5,8-tetraene;
6-methyl-7-phenyl-5,7,13-triazatetracyclo[9.3.1.$0^{2.10}$.$0^{4,8}$]pentadeca-2(10),3,5,8-tetraene;
6,7-dimethyl-5,8,14-triazatetracyclo[10.3.1.$0^{2,11}$.$0^{4,9}$]hexadeca-2(11),3,5,7,9-pentaene;
5,8,14-triazatetracyclo[10.3.1.$0^{2,11}$.$0^{4,9}$]hexadeca-2(11),3,5,7,9-pentaene;
14-methyl-5,8,14-triazatetracyclo[10.3.1.$0^{2,11}$.$0^{4,9}$]hexadeca-2(11),3,5,7,9-pentaene;
5-oxa-7,13-diazatetracyclo[9.3.1.$0^{2,10}$.$0^{4,8}$]pentadeca-2(10),3,6,8-tetraene;
6-methyl-5-oxa-7,13-diazatetracyclo[9.3.1.$0^{2,10}$.$0^{4,8}$]pentadeca-2(10),3,6,8-tetraene;
4-chloro-10-azatricyclo[6.3.1.$0^{2,7}$]dodeca-2(7),3,5-triene;
10-azatricyclo[6.3.1.$0^{2,7}$]dodeca-2(7),3,5-trien-4-yl cyanide;
1-(10-azatricyclo[6.3.1.$0^{2,7}$]dodeca-2(7),3,5-trien-4-yl)-1-ethanone;
10-azatricyclo[6.3.1.$0^{2,7}$]dodeca-2(7),3,5-trien-4-ol;
7-methyl-5-oxa-6,13-diazatetracyclo[9.3.1.$0^{2,10}$.$0^{4,8}$]pentadeca-2,4(8),6,9-tetraene;
4,5-dichloro-10-azatricyclo[6.3.1.$0^{2,7}$]dodeca-2(7),3,5-triene;
11-azatricyclo[7.3,1.$0^{2,7}$]trideca-2(7),3,5-triene-5-carbonitrile;
1-[11-azatricyclo[7.3.1.$0^{2,7}$]trideca-2(7),3,5-trien-5-yl]-1-ethanone;
1-[11-azatricyclo[7.3.1.$0^{2,7}$]trideca-2(7),3,5-trien-5-yl]-1-propanone;
4-fluoro-11-azatricyclo[7.3.1.$0^{2,7}$]trideca-2(7),3,5-triene-5-carbonitrile;
5-fluoro-11-azatricyclo[7.3.1.$0^{2,7}$]trideca-2(7),3,5-triene-4-carbonitrile;
6-methyl-7-thia-5,14-diazatetracyclo[10.3.1.$0^{2,10}$.$0^{4,8}$]hexadeca-2(10),3,5,8-tetraene;
6-methyl-5,7,14-triazatetracyclo[10.3.1.$0^{2,10}$.$0^{4,8}$]hexadeca-2(10),3,5,8-tetraene;
6,7-dimethyl-5,7,14-triazatetracyclo[10.3.1.$0^{2,10}$.$0^{4,8}$]hexadeca-2(10),3,5,8-tetraene;
5,7,14-triazatetracyclo[10.3.1.$0^{2,10}$.$0^{4,8}$]hexadeca-2(10),3,5,8-tetraene;
5,6-dimethyl-5,7,14-triazatetracyclo[10.3.1.$0^{2,10}$.$0^{4,8}$]hexadeca-2(10),3,6,8-tetraene;
5-methyl-5,7,14-triazatetracyclo[10.3.1.$0^{2,10}$.$0^{4,8}$]hexadeca-2(10),3,6,8-tetraene;
6-(trifluoromethyl)-7-thia-5,14-diazatetracyclo[10.3.1.$0^{2,10}$.$0^{4,8}$]hexadeca-2(10),3,5,8-tetraene;
5,8,15-triazatetracyclo[11.3.1.$0^{2,11}$.$0^{4,9}$]heptadeca-2(11),3,5,7,9-pentaene;
7-methyl-5,8,15-triazatetracyclo[11.3.1.$0^{2,11}$.$0^{4,9}$]heptadeca-2(11),3,5,7,9-pentaene;
6-methyl-5,8,15-triazatetracyclo[11.3.1.$0^{2,11}$.$0^{4,9}$]heptadeca-2(11),3,5,7,9-pentaene;
6,7-dimethyl-5,8,15-triazatetracyclo[11.3.1.$0^{2,11}$.$0^{4,9}$]heptadeca-2(11),3,5,7,9-pentaene;
7-oxa-5,14-diazatetracyclo[10.3.1.$0^{2,10}$.$0^{4,8}$]hexadeca-2(10),3,5,8-tetraene;
6-methyl-7-oxa-5,14-diazatetracyclo[10.3.1.$0^{2,10}$.$0^{4,8}$]hexadeca-2(10),3,5,8-tetraene;

5-methyl-7-oxa-6,14-diazatetracyclo[10.3.1.$0^{2,10}$.0.$^{4,8}$]hexadeca-2(10),3,5,8-tetraene;
6-methyl-5-oxa-7,14-diazatetracyclo[10.3.1.$0^{2,10}$.$0^{4,8}$]hexadeca-2(10),3,6,8-tetraene;
7-methyl-5-oxa-6,14-diazatetracyclo[10.3.1.$0^{2,10}$.$0^{4,8}$]hexadeca-2(10),3,6,8-tetraene;
4,5-difluoro-11-azatricyclo[7.3.1.$0^{2,7}$]trideca-2(7),3,5-triene;
4-chloro-5-fluoro-11-azatricyclo[7.3.1.$0^{2,7}$]trideca-2(7),3,5-triene;
5-chloro-4-fluoro-11-azatricyclo[7.3.1.$0^{2,7}$]trideca-2(7),3,5-triene;
4-(1-ethynyl)-5-fluoro-11-azatricyclo[7.3.1.$0^{2,7}$]trideca-2(7),3,5-triene;
5-(1-ethynyl)-4-fluoro-11-azatricyclo[7.3.1.$0^{2,7}$]trideca-2(7)3,5-triene;
56-difluoro-11-aza-tricyclo[7.3.1.$0^{2,7}$]trideca-2,4,6-triene;
6-trifluoromethyl-11-aza-tricyclo[7.3.1.$0^{2,7}$]trideca-2,4,6-triene;
6-methoxy-11-aza-tricyclo[7.3.1.$0^{2,7}$]trideca-2(7),3,5-triene;
11-aza-tricyclo[7.3.1.$0^{2,7}$]trideca-2(7),3,5-trien-6-ol;
6-fluoro-11-aza-tricyclo[7.3.1.$0^{2,7}$]trideca-2(7),3,5-triene;
11-aza-tricyclo[7.3.1.$0^{2,7}$]trideca-2(7),3,5-trien-5-ol;
4-nitro-11-aza-tricyclo[7.3.1.$0^{2,7}$]trideca-2(7),3,5-triene;
5-nitro-11-aza-tricyclo[7.3.1.$0^{2,7}$]trideca-2(7),3,5-triene;
5-fluoro-11-aza-tricyclo[7.3.1.$0^{2,7}$]trideca-2(7),3,5-triene; and
6-hydroxy-5-methoxy-11-aza-tricyclo[7.3.1.$0^{2,7}$]trideca-2(7),3,5-triene and

their pharmaceutically acceptable salts and their optical isomers.

Preferably, the nicotine receptor partial agonist is selected from:

9-bromo-1 ,2,3,4,5,6-hexahydro-1,5-methano-pyrido[1,2-a][1,5]diazocin-8-one;
9-chloro-1,2,3,4,5,6-hexahydro-1 ,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1 ,5-methano-pyrido[1,2a][1,5]diazocin-8-one;
9-cyano-1,2,3,4,5,6-hexahydro-1,5-methano-pyrido[1,2a][1,5]diazocin-8-one;
9-carbomethoxy-1,2,3,4,5,6-hexahydro-1 ,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
6-methyl-5-thia-5-dioxa-6,13-diazatetracyclo[9.3.1.$0^{2,10}$.$0^{4,8}$]pentadeca-2(10),3,8-triene;
4-fluoro-10-aza-tricyclo[6.3.1.$0^{2,7}$]dodeca-2(7),3,5-triene;
4-trifluoromethyl-10-aza-tricyclo[6.3.1.$0^{2,7}$]dodeca-2(7),3,5-triene;
4-nitro-10-azatricyclo[6.3.1.$0^{2,7}$]dodeca-2(7),3,5-triene;
6-methyl-5,7,13-triazatetracyclo[9.3.1.$0^{2,10}$.$0^{4,8}$]pentadeca-2(10),3,5,8-tetraene;
6,7-dimethyl-5,8,14-triazatetracyclo[10.3.1.$0^{2,11}$.$0^{4,9}$]hexadeca-2(11),3,5,7,9-pentaene;
5,8,14-triazatetracydo[10.3.1.$0^{2,11}$.$0^{4,9}$]hexadeca-2(11 ),3,5,7,9-pentaene;
5-oxa-7,13-diazatetracyclo[9.3.1.$0^{2,10}$.$0^{4,8}$]pentadeca-2(10),3,6,8-tetraene;
6-methyl-5-oxa-7,13-diazatetracyclo[9.3.1.$0^{2,10}$.$0^{4,8}$]pentadeca-2(10),3,6,8-tetraene;
10-azatricyclo[6.3.1.$0^{2,7}$]dodeca-2(7),3,5-trien-4-yl cyanide;
1-(10-azatricyclo[6.3.1.$0^{2,7}$]dodeca-2(7),3,5-trien-4-yl)-1-ethanone;
11-azatricyclo[7.3.1.$0^{2,7}$]trideca-2(7),3,5-triene-5-carbonitrile;
1-[11-azatricyclo[7.3.1.$0^{2,7}$]trideca-2(7),3,5-trien-5-yl]-1-ethanone;
1-[11-azatricyclo[7.3.1.$0^{2,7}$]trideca-2(7),3,5-trien-5-yl]-1-propanone;
4-fluoro-11-azatricyclo[7.3.1.$0^{2,7}$]trideca-2(7),3,5-triene-5-carbonitrile;
5-fluoro-11-azatricyclo[7.3.1.$0^{2,7}$]trideca-2(7),3,5-triene-4-carbonitrile;
6-methyl-7-thia-5,14-diazatetracyclo[10.3.1.$0^{2,10}$.$0^{4,8}$]hexadeca-2(10),3,5,8-tetraene;
6-methyl-5,7,14-triazatetracyclo[10.3.1.$0^{2,10}$.$0^{4,8}$]hexadeca-2(10),3,5,8-tetraene;
6,7-dimethyl-5,7,14-triazatetracyclo[10.3.1.$0^{2,10}$.$0^{4,8}$]hexadeca-2(10),3,5,8-tetraene;
6-methyl-7-oxa-5,14-diazatetracyclo[10.3.1.$0^{2,10}$.$0^{4,8}$]hexadeca-2(10),3,5,8-tetraene;
6-methyl-5-oxa-7,14-diazatetracyclo[10.3.1.$0^{2,10}$.$0^{4,8}$]hexadeca-2(10),3,6,8-tetraene;
5,6-difluoro-11-aza-tricyclo[7.3.1.$0^{2,7}$]trideca-2,4,6-triene;
6-trifluoromethyl-11-aza-tricyclo[7.3.1.$0^{2,7}$]trideca-2,4,6-triene;
6-methoxy-11 -aza-tricyclo[7.3.1.$0^{2,7}$]trideca-2(7),3,5-triene;
6-fluoro-11-aza-tricyclo[7.3.1.$0^{2,7}$]trideca-2(7),3,5-triene; and

11-aza-tricyclo[7.3.1.$0^{2,7}$]trideca-2(7),3,5-trien-5-ol;
and the pharmaceutically acceptable salts stereoisomers (including optical isomers), solvates and hydrates of the foregoing compounds.

Examples of $D_2$ receptor agonists that can be used in the pharmaceutical compositions of this invention include, but are not limited to, pergolide (Permax), bromocriptine (Parlodel), ropinirole (Requip), and pramipexole (Mirapex), pemoline (Cylert), cabergoline (Dostinex), amphetamine (Adderall), dextroamphetamine (Dexedrine) and methylphenidate (Ritalin) and their pharmaceutically acceptable salts.

Examples of $5HT_{1A}$ receptor agonists that can be used in the pharmaceutical compositions of this invention include, but are not limited to: (a) sunepitron, and other bis-azabicyclic compounds disclosed in U.S. patent 5,122,525 and their pharmaceutically acceptable salts, (b) buspirone (Buspar) (c) gepirone; (c) ipsapirone; and (d) and flesinoxan.

Examples of $\alpha_2$ adrenergic receptor antagonists that can be used in the compositions of this invention include, but are not limited to, yohimbine, idazoxan and clonidine (Catapres) and their pharmaceutically acceptable salts.

Examples of $\alpha_1$ adrenergic receptor agonists that can be used in the compositions of this invention include, but are not limited to, modafinil (Provigil) and their pharmaceutically acceptable salts.

Examples of norepinephrine re-uptake inhibitors (NEURI) that can be used in the compositions of this invention include, but are not limited to tomoxetine, bupropion (Wellbutrin), and venlafaxine (Effexor) and their pharmaceutically acceptable salts.

The nicotine receptor partial agonist and anti-ADHD agent can be administered substantially simultaneously.

The term "treating" as used herein, refers to reversing, alleviating, inhibiting or slowing the progress of, or preventing the disorder or condition to which such term applies, or one or more symptoms of such disorder or condition. The term "treatment", as used herein, refers to the act of treating, as "treating" is defined immediately above.

## Detailed Description of the Invention

In combination with the NRPA, the invention includes an anti-ADHD agent or a pharmaceutically acceptable salt of compounds including, but not limited to, $5HT_{1A}$ receptor agonists (e.g. buspirone (Buspar), gepirone, ipsapirone, flesinoxan, sunepitron, and bis-azabicyclo compounds disclosed in U.S. Patent No. 5,122,525); $D_2$ receptor agonists including but not limited to, pergolide (Permax), bromocriptine (Parlodel), ropinirole (Requip), and pramipexole (Mirapex), pemoline (Cylert), cabergoline (Dostinex), amphetamine (Adderall), dextroamphetamine (Dexedrine) and methylphenidate (Ritalin); $\alpha_2$ adrenergic receptor antagonists include but are not limited to, yohimbine, idazoxan and clonidine (Catapres); $\alpha_1$ adrenergic receptor agonists include but are not limited to, modafinil (Provigil); norepinephrine re-uptake inhibitors (NEURI) include but are not limited to tomoxetine, bupropion (Wellbutrin), and venlafaxine (Effexor).

A nicotine partial agonist combined with an anti-ADHD agent will relieve symptoms of ADHD while reducing the level of undesirable side effects. Nicotine has long been appreciated to have anti-ADHD properties, but its use has been limited by a poor spectrum of activity, side effects, and less efficacy than other anti-ADHD agents. This may be due to lack of specificity of nicotine for neuromuscular, ganglionic, and central nervous system receptors. The development of nicotine partial agonists with specific receptor subtype affinities is an approach to potentially reduce side effects and enhance efficacy.

The particular NRPA compounds listed above, which can be employed in the methods and pharmaceutical compositions of this invention, can be made by processes known in the chemical arts, for example by the methods described in WO 9818798 A1, WO 9935131-A1 and WO9955680-A1 and incorporated by reference herein. Some of the preparation methods useful for making the compounds of this invention may require protection of remote functionality (i.e., primary amine, secondary amine, carboxyl). The need for such protection will vary depending on the nature of the remote functionality and the conditions of the preparation methods. The need for such protection is readily determined by one skilled in the art, and is described in examples carefully described in the above cited applications. The starting materials and reagents for the NRPA compounds employed in this invention are also readily available or can be easily synthesized by those skilled in the art using conventional methods of organic synthesis. Some of the compounds used herein are related to, or are derived from compounds found in nature and accordingly many such compounds are commercially available or are reported in the literature or are easily prepared from other commonly available substances by methods which are reported in the literature.

Some of the NRPA compounds employed in this invention are ionizable at physiological conditions. Thus, for example some of the compounds of this invention are acidic and they form a salt with a pharmaceutically acceptable cation. The use of all such salts are within the scope of the pharmaceutical compositions and methods this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, some of the NRPA compounds employed in this invention are basic, and they form a salt with a pharmaceutically acceptable acid. All such salts are within the scope of this invention and they can be prepared by conventional methods. For example, they can be prepared simply by contacting the basic and acidic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

The utility of the NRPA compounds employed in the present invention as medicinal agents in the treatment of ADHD mammals (e.g. humans) is demonstrated by the activity of the compounds of this invention in conventional assays and, in particular the assays described below. Such assays also provide a means whereby the activities of the compounds of this invention can be compared between themselves and with the activities of other known compounds. The results of these comparisons are useful for determining dosage levels in mammals, including humans, for the treatment of such diseases.

Administration of the compositions of this invention can be via any method which delivers a compound of this invention systemically and/or locally. These methods which include oral routes and transdermal routes, etc. Generally, the compounds of this invention are administered orally, but parenteral administration may be utilized (e.g., intravenous, intramuscular, subcutaneous or intramedullary). The two different compounds of this invention can be co-administered simultaneously or sequentially in any order, or single pharmaceutical composition comprising a NRPA as described above and an analgesic agent as described above in a pharmaceutically acceptable carrier can be administered.

Procedures

Receptor binding assay: The effectiveness of the active compounds in suppressing nicotine binding to specific receptor sites is determined by the following procedure which is a modification of the methods of Lippiello, P. M. and Fernandes, K. G. (in The Binding of L-[$^3$H]Nicotine To A Single Class of High-Affinity Sites in Rat Brain Membranes, Molecular Pharm., 29, 448-54, (1986)) and Anderson, D. J. and Arneric, S. P. (in Nicotinic Receptor Binding of $^3$H-Cytisine, $^3$H-Nicotine and $^3$H-Methylcarmbamylcholine In Rat Brain, European J. Pharm., 253, 261-67 (1994)). Male Sprague-Dawley rats (200-300 g) from Charles River were housed in groups in hanging stainless steel wire cages and were maintained on a 12 hour light/dark cycle (7 a.m.-7 p.m. light period). They received standard Purina Rat Chow and water *ad libitum.* The rats were killed by decapitation. Brains were removed immediately following decapitation. Membranes were prepared from brain tissue according to the methods of Lippiello and Fernandez (Molec Pharmacol, 29, 448-454, (1986) with some modifications. Whole brains were removed, rinsed with ice-cold buffer, and homogenized at 0° in 10 volumes of buffer (w/v) using a Brinkmann Polytron™, setting 6, for 30 seconds. The buffer consisted of 50 mM Tris HCl at a pH of 7.5 at room temperature. The homogenate was sedimented by centrifugation (10 minutes; 50,000 x g; 0° to 4°C). The supernatant was poured off and the membranes were gently resuspended with the Polytron and centrifuged again (10 minutes; 50,000 x q; 0° to 4°C. After the second centrifugation, the membranes were resuspended in assay buffer at a concentration of 1.0 g/100 mL. The composition of the standard assay buffer was 50 mM Tris HCl, 120 mM NaCl, 5 mM KCl, 2 mM $MgCl_2$, 2 mM $CaCl_2$ and has a pH of 7.4 at room temperature.

Routine assays were performed in borosilicate glass test tubes. The assay mixture typically consisted of 0.9 mg of membrane protein in a final incubation volume of 1.0 mL. Three sets of tubes were prepared wherein the tubes in each set contained 50 μL of vehicle, blank, or test compound solution, respectively. To each tube was added 200 μL of [$^3$H]-nicotine in assay buffer followed by 750 μL of the membrane suspension. The final concentration of nicotine in each tube was 0.9 nM. The final concentration of cytisine in the blank was 1 μM. The vehicle consisted of deionized water containing 30μL of 1 N acetic acid per 50 mL of water. The test compounds and cytisine were dissolved in vehicle. Assays were initiated by vortexing after addition of the membrane suspension to the tube. The samples were incubated at 0° to 4°C in an iced shaking water bath. Incubations were terminated by rapid filtration under vacuum through Whatman GF/B™ glass fiber filters using a Brandel™ multi-manifold tissue harvester. Following the initial filtration of the assay mixture, filters were washed two times with ice-cold assay buffer (5 m each). The filters were then placed in counting vials and mixed vigorously with 20 ml of Ready Safe™ (Beckman) before quantification of radioactivity. Samples were counted in a LKB Wallach Rackbeta™ liquid scintillation counter at 40-50% efficiency. All determinations were in triplicate.

Calculations: Specific binding (C) to the membrane is the difference between total binding in the samples containing vehicle only and membrane (A) and non-specific binding in the samples containing the membrane and cytisine (B), i.e.,

Specific binding = (C) = (A) - (B).

Specific binding in the presence of the test compound (E) is the difference between the total binding in the presence of the test compound (D) and non-specific binding (B), i.e., (E) = (D) - (B).

% Inhibition = (1-((E)/(C)) times 100.

The compounds of the invention that were tested in the above assay exhibited $IC_{50}$ values of less than 10 μM. D$_2$ receptor binding assay: LTK$^-$ cells expressing the human $D_2$ long ($D_{2L}$) receptor are grown (T-175 flasks) in D-glucose containing minimal essential media (DMEM, Gibco) supplemented with 10% fetal bovine serum (FBS). The cells are dislodged with 5 mM EDTA in PBS and homogenized in 50 mM Tris HCl (pH 7.4) with 5mM $MgSO_4$, using a Brinkman Polytron at setting 6 for 20 sec. Membranes are recovered after multiple rounds of separation by centrifugation and resuspension in fresh ice-cold buffer. The tissue (~ 2 mg tissue, wet weight) is added to test tubes containing incubation buffer (50 mM Tris HCl, 120 mM NaCl, 2 mM $MgCl_2$, 5 mM KCl, 5 mM $CaCl_2$, pH 7.2), various concentrations of test drug, and [$^3$H]-spiperone (0.06 nM final concentration, Amersham, Arlington Heights IL). Non-specific binding is determined in the presence of 2 uM (+)-butaclamol. After 45 min at 30°C, incubations are terminated by rapid filtration through Whatman GF/B filters using a Brandel cell harvester. The membranes are washed using 3 X 4 ml of ice-cold buffer and membrane-bound ligand is determined by liquid scintillation counting of the filters in Ready-Safe scintillation cocktail (for tritiated ligands). The $K_d$ (0.06 nM) for the radioligand is determined previously by saturation analysis and used to calculate apparent $K_i$'s by means of the Cheng-Prusoff equation.

Acetylcholinesterase inhibitor Protocol

Inhibition of Acetylcholinesterase (AChE) and Butyrylcholinesterase (BuChE). The method of Ellman, GL.; Courtney, K.D.; Andres, V., Jr.; Featherstone, R.M. A New and Rapid Colorimetric Determination of Acetylcholinesterase Activity. *Biochem. Pharmacol*. 1961, 7, 88-95 was followed. The assay solution consists of a 0.1 M sodium phosphate buffer, pH 8.0, with the addition of 100 μM tetraisopropypyrophosphoramide (*iso*-OMPA), 100 μM 5,5'-dithiobis (2-nitrobenzoic acid) (DTNB), 0.02 units/mL AChE (Sigma Chemical Col, from human erythrocytes) and 200 μM acetylthiocholine iodide. The final assay volume was 0.25 mL. Test compounds were added to the assay solution prior to enzyme addition, whereupon a 20-min preincubation period with enzyme was followed by addition of substrate. Changes in absorbance at 412 nM were recorded for 5 min. The reaction rates were compared, and the percent inhibition due to the presence of test compounds was calculated.

Inhibition of butyrylcholinesterase was measured as described above for AChE by omitting addition of *iso*-OMPA and substitution 0.02 units/mL of BuChE (Sigma Chemical Co., from horse serum) and 200 μM butyrylthiocholine for enzyme and substrate, respectively.

*In vivo* Microdialysis. Male Sprague-Dawley rats were implanted in the corpus striatum with guide cannulae and dialysis probes (Bioanalytical Systems, West Lafayette, IN) and superfused at a rate 3 mL/minute. The dialysis fluid was a Ringer's buffer (pH 7.2) containing 500 nM physostigmine to reduce degradation of Ach by AChE. Fractions (60 μl) were collected every 20 minutes for 2 hours before drug administration and for 3 hours following oral administration of drug. Samples (50 μl) were used directly for HPLC analysis of Ach content as described above. Basal Ach release was defined as the average Ach content in the three fractions just prior to drug administration. Ach content in all fractions was converted to a percentage of these basal control values.

The agonist or antagonist activity of a compound at the $D_2$ receptor can be determined using the following three assays.

(1) Human $D_2$ receptor modulation of cAMP formation in $GH_4C_1$ cells

$GH_4C_1$ cells, derived from rat pituitary, expressing either the long or short forms of the human $D_2$ receptor, are grown to confluence in (HAM) F-10 Nutrient Mixture (Gibco) supplemented with 10% FBS and 2 mM I-glutamine and 10 U/ml penicillin-streptomycin in T-175 flasks. The cells are dislodged with 5 mM ethylenediamine tetraacetic acid (EDTA) in phosphate buffered saline (PBS) and resuspended in PBS containing 5 mM $MgCl_2$, 30 mM hydroxyethylpiperizine-N-ethanesulfonic acid (HEPES), and 50 mM isobutyl methyl xanthine (IBMX). Cells (~200,000/tube) are exposed to 5 mM forskolin, 100 nM quinpirole or forskolin plus quinpirole plus antagonist for 11 minutes. In experiments with antagonists, cells are exposed to the antagonists 11 minutes prior to quinpirole challenge. To judge agonist activity, the effect of a compound on forskolin stimulated cAMP accumulation is tested in the absence of the agonist quinpirole. The reaction is terminated with the addition of 6N perchloric acid, and samples are neutralized with 5N potassium hydroxide and 2 M Tris buffer. Cyclic AMP levels are measured using a commercially available competitive binding kit (Amersham). $IC_{50}$ values are calculated by linear regression analysis of the concentration-response curves. Apparent $K_i$ values are calculated using the equation: $K_i = IC_{50}/(1 + [agonist]/[agonist\ EC_{50}])$.

(2) Electrophysiology in Rat Brain Slices

Male Sprague-Dawley rats (200-250 gm, Charles River Laboratories, Wilmington, MA) are lightly anesthetized with halothane, decapitated and the brains quickly removed to ice-cold, oxygenated medium (95% $O_2$/5% $CO_2$; 124 mM NaCl, 2 mM KCl, 1.25 mM $NaH_2PO_4$, 26 mM $NaHCO_3$, 10 mM d-glucose, 2 mM $MgSO_4 \cdot 7H_2O$ and 2 mM $CaCl_2$; pH 7.4). The ventral tegmental area is blocked and glued using cyanoacrylate to the stage of a Lancer Vibratome (Series 1000) filled with ice cold medium. Coronal slices (350 µ) are cut and placed in oxygenated medium (22°C) for 1 hour prior to recording. For recording, slices are placed on a nylon net in a recording chamber where they are completely submerged in continually flowing medium at 35°C (~1 ml/min). All drugs are applied by switching the perfusion medium to a solution containing the drug. Spontaneous extracellular action potentials are recorded using 0.9% saline-filled glass pipettes (6-8 MW). Firing rates are plotted on-line in bins of 10 or 20 seconds and alterations in firing rate are calculated using average rates over 2 min epochs before and after drug application. Concentration-response curves are constructed and analyzed by linear regression.

(3) Microdialysis

I shaped concentric microdialysis probes are constructed out of dialysis fiber (molecular weight cut off of 18,000, 300 um o.d., Hospal, The Netherlands) occluded at one end with epoxy resin and attached to fused silica microtubing. The probes, 9 mm long with 2 mm length of exposed dialysis membrane are implanted into the nucleus accumbens (AP 1.7 , ML -1.2 ,DV -8.0) of male Sprague Dawley rats (300-350g) anesthetized with ketamine (75 mg/kg) and xylazine (10 mg/kg). Following surgery, rats are placed in perspex cages inside insulation boxes and the probe inlets connected via flexible PEEK tubing through a dual channel fluid swivel system to a CMA/100 microinfusion pump (CMA/Microdialysis, Acton, MA). The probe is perfused overnight with artificial cerebrospinal fluid (147 mM $NaCl_2$, 2.7 mM KCl, 1.3 mM $CaCl_2$, 1.0 mM $MgCl_2$ and 0.1 mM ascorbic acid) at 0.5 ml/min. The next day, an experiment is started by increasing the flow to 1.5 ml/min and connecting the probe outlet with PEEK tubing to a 30 ml sample loop in a DECADE electrochemical detector (ANTEC, Leiden, The Netherlands). Microdialysis samples (30 ml) are collected on-line and automatically injected onto the column every 20 or 25 min. Analytes are separated at 35°C over a BDS Hypersil $C_{18}$ 3 m column (150 X3 mm) by reverse phase HPLC using a 75 mM potassium phosphate mobile phase of pH 5.0, containing 0.8 mM octanesulfonate, 8% methanol, 3 mM triethylamine and delivered at a flow rate of 0.35 ml/min by an ESA 580 pump. Amperometric detection of dopamine is performed using a glassy carbon electrode of the DECADE detector set at 550 mV vs Ag/AgCl. Extracellular levels of dopamine are quantified by comparing peak heights with those of standards.

After obtaining a stable baseline (5-7 samples collected every 20 or 25 min) drugs are administered and release of DA monitored for 4 to 7 hours. Dialysate concentrations are expressed as a percentage of baseline. Dialysate concentrations of DA are not corrected for recovery across the dialysis fiber. To determine whether each dose of drug has a significant effect on nucleus accumbens DA release multivariate analysis of variance with repeated measures over time is performed using SuperANova software (Abacus Concepts, Inc. Berkeley CA.)

5-$HT_{1A}$ receptor procedures: The agonist and antagonist activities of a particular compound of the invention at 5-$HT_{1A}$ receptors can be determined using a single saturating concentration according to the following procedure. Male Hartley guinea pigs are decapitated and 5-$HT_{1A}$ receptors are dissected out of the hippocampus. The individual tissues are homogenized in 5 mM HEPES buffer containing 1 mM EGTA (pH 7.5) using a hand-held glass-Teflon® homogenizer and centrifuged at 35,000 x g for 10 minutes at 4°C. The pellets are resuspended in 100 mM HEPES buffer containing 1 mM EGTA (pH 7.5) to a final protein concentration of 20 mg (hippocampus) or 5 mg (substantia nigra) of protein per tube. The following agents are added so that the reaction mix in each tube contained 2.0 mM $MgCl_2$, 0.5 mM ATP, 1.0 mM cAMP, 0.5 mM IBMX, 10 mM phosphocreatine, 0.31 mg/mL creatine phosphokinase, 100 mM GTP and 0.5-1 microcuries of [$^{32}$P]-ATP (30 Ci/mmol: NEG-003-New England Nuclear). Incubation is initiated by the addition of tissue to siliconized microfuge tubes (in triplicate) at 30°C for 15 minutes. Each tube receives 20 mL tissue, 10 mL drug or buffer (at 10X final concentration), 10mL 32 nM agonist or buffer (at 10X final concentration), 20mL forskolin (3 mM final concentration) and 40 mL of the preceding reaction mix. Incubation is terminated by the addition of 100 mL 2% SDS, 1.3 mM cAMP, 45 mM ATP solution containing 40,000 dpm [$^{3}$H]-cAMP (30 Ci/mmol: NET-275 - New England Nuclear) to monitor the recovery of cAMP from the columns. The separation of [$^{32}$P]-ATP and [$^{32}$P]-cAMP is accomplished using the method of Salomon et al., Analytical Biochemistry, **1974,** 58, 541-548. Radioactivity is quantified by liquid scintillation counting. Maximal inhibition is defined by 10 mM (R)-8-OH-DPAT for 5-$HT_{1A}$ receptors. Percent inhibition by the test compound is then calculated in relation to the inhibitory effect of (R)-8-OH-DPAT. The reversal of agonist induced inhibition of forskolin-stimulated adenylate cyclase activity is calculated in relation to the 32 nM agonist effect.

Anti-ADHD behavioral assays: The ability of a particular compound to exhibit an anti-ADHD effect in nonhuman primates can be determined using the procedures described by Greenemyre et al., Ann. Neurol., 35:655-661, 1994,

and Klockgether et al., Ann. Neurol., 30:717-723, 1991.

The amount and timing of compounds administered will, of course, be based on the judgement of the prescribing physician. Thus, because of patient to patient variability, the dosages given below are a guideline and the physician may titrate doses of the agent to achieve the activity that the physician considers appropriate for the individual patient. In considering the degree of activity desired, the physician must balance a variety of factors such as cognitive function, age of the patient, presence of preexisting disease, as well as presence of other diseases (e.g., cardiovascular). The following paragraphs provide preferred dosage ranges for the various components of this invention (based on average human weight of 70 kg).

In general, an effective dosage for the NRPA in the range of 0.001 to 200 mg/kg/day, preferably 0.01 to 10.0 mg/kg/day.

For use in treating ADHD in a human subject, the anti-ADHD agent or a pharmaceutically-acceptable salt thereof, is administered in an amount of about 0.001-300 mg/day, in single or divided daily doses. In particular cases, dosages outside that range are prescribed at the discretion of the attending physician. The preferred route of administration is generally oral, but parenteral administration (e.g., intramuscular, intravenous, intrademal) will be preferred in special cases, e.g., where oral absorption is impaired as by disease, or the patient is unable to swallow.

A controlled-release formulation can be employed instead that is administered once per day. The compounds used in the present invention are generally administered in the form of pharmaceutical compositions comprising at least one of the NRPAs described in United States Patent or a salt thereof, together with a pharmaceutically acceptable vehicle or diluent. Such compositions are generally formulated in a conventional manner utilizing solid or liquid vehicles or diluents as appropriate to the mode of desired administration: for oral administration, in the form of tablets, hard or soft gelatin capsules, suspension, granules, powders and the like; and, for parenteral administration, in the form of injectable solutions or suspensions, and the like.

This invention relates both to methods of treating ADHD in which the two or three active agents employed are administered together, as part of the same pharmaceutical composition, as well as to methods in which these active agents are administered separately as part of an appropriate dose regimen designed to obtain the benefits of the combination therapy. The appropriate dose regimen, the amount of each dose administered, and specific intervals between doses of each active agent will depend upon the subject being treated, how well the drugs are tolerated and the severity of the condition. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The combination methods of this invention include methods wherein the desired combined activities are present in one compound or pharmaceutically acceptable salt. The pharmaceutical compositions of this invention that exhibit more than one pharmaceutical activity (e.g., $5HT_{1A}$ agonism and $D_2$ agonism) include those pharmaceutical compositions wherein all the desired pharmaceutical activities are present in one compound or pharmaceutically acceptable salt.

The $\alpha_2$ adrenergic receptor ligands, $\alpha_1$ adrenergic receptor ligands, $D_2$ receptor agonists, NEURI, $5HT_{1A}$ receptor agonists, and cholinesterase inhibitors that are employed in the pharmaceutical compositions and methods of this invention are hereinafter also referred to as "therapeutic agents". The therapeutic agents can be administered via either the oral or parenteral route daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, i.e., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic compounds of this invention, when administered separately (i.e., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk

sugar as well as high molecular weight polyethylene glycols. When aqueous suspensions and/or elixirs are desired for oral administration, the active ingredient, may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

The following example is intended only to illustrate the invention, and not to be interpreted as limiting its scope.

For bromocriptine (Parlodel) the range is about 0.001 to about 0.15 mg/kg/day
For methylphenidate (Ritalin) the range is about 0.01 to about 0.85 mg/kg/day
For pergolide (Permax) the range is about 0.0007 to about 0.07 mg/kg/day
For ropinirole (Requip) the range is about 0.01 to about 0.043 mg/kg/day
For pramipexole (Mirapex) the range is about 0.005 to about 0.064 mg/kg/day
For pemoline (Cylert) the range is about 0.1 to about 1.6 mg/kg/day
For amphetamine (Adderall) the range is about 0.05 to about 0.6 mg/kg/day
For dextroamphetamine (Dexedrine) the range is about 0.07 to about 0.85 mg/kg/day
For cabergoline (Dostinex) the range is about 0.0036 mg/kg to about 0.0143 mg/kg twice per week
For buspirone (Buspar) the range is about 0.07 to about 0.85 mg/kg/day
For sunepitron the range is about 0.01 to about 0.3 mg/kg/day
For gepirone the range is about 0.05 to about 1.4 mg/kg/day
For ipsapirone the range is about 0.05 to about 0.4 mg/kg/day
For flesinoxan the range is about 0.001 to about 0.07 mg/kg/day
For yohimbine the range is about 0.01 to about 0.2 mg/kg/day
For idazoxan the range is about 0.07 to about 2.0 mg/kg/day
For clonidine (Catapres) the range is about 0.001 to about 0.034 mg/kg/day
For tomoxetine the range is about 0.1 to about 1.1 mg/kg/day
For modafinil (Provigil) the range is about 1.0 to about 5.7 mg/kg/day
For bupropion (Wellbutrin) the range is about 1.0 to about 4.3 mg/kg/day
For venlafaxine (Effexor) the range is about 0.15 to about 5.4 mg/kg/day
For donepezil (Aricept™) the range is about 0.01 to about 0.15 mg/kg/day
For tacrine (Cognex™) the range is about 0.1 to about 2.3 mg/kg/day
For rivastigmine (Exelon™) the range is about 0.1 to about 0.1 mg/kg/day
For physostigmine (Synapton) the range is about 0.01 to about 0.4 mg/kg/day
For galanthamine (Reminyl) the range is about 0.05 to about 0.5 mg/kg/day
For metrifonate (Promem) the range is about 0.1 to about 5.0 mg/kg/day
For neostigmine (Prostigmin) the range is about 0.1 to about 5.0 mg/kg/day
For icopezil (NOTE this = CP-118,954) the range is about 0.001 to about 0.01 mg/kg/day

## Claims

**1.** A pharmaceutical composition for the treatment of Attention Deficit Hyperactivity Disorder Comprising

(a) a nicotine receptor partial agonist or a pharmaceutically acceptable salt thereof;
(b) an $\alpha_2$ adrenergic receptor ligand, an $\alpha_1$ adrenergic receptor ligand, a $D_2$ receptor agonist, a NEURI, a $5HT_{1A}$ receptor agonist, and/or a cholinesterase inhibitor or a pharmaceutically acceptable salt thereof; and
(c) a pharmaceutically acceptable carrier;

wherein the active agents "a" and "b" above are present in amounts that render the composition effective in treating Attention Deficit Hyperactivity Disorder (ADHD).

**2.** A pharmaceutical composition according to Claim 1, wherein the $\alpha_2$ adrenergic receptor antagonist is $\alpha_2$ a presynaptic $\alpha_2$ adrenergic antagonist or a postynaptic $\alpha_2$ adrenergic receptor agonist.

**3.** The pharmaceutically composition according to Claim 1, wherein said nicotine receptor partial agonist is selected from:

9-bromo-1,2,3,4,5,6-hexahydro-1,5-methano-pyrido[1,2-a][1,5]diazocin-8-one;
9-chloro-1,2,3,4,56-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1,5]diazocin-8-one;
9-(2-propyl)- 1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one;
9-(3,5-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(2,5-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.0$^{2,10}$.0$^{4,8}$]pentadeca-2(10),3,8-triene;
5-oxo-6,13-diazatetracyclo[9.3.1.0$^{2,10}$.0$^{4,8}$]pentadeca-2(10),3,8-triene;
6-oxo-5,7,13-triazatetracyclo[9.3.1.0$^{2,10}$.0$^{4,8}$]pentadeca-2(10),3,8-triene;
4,5-difluoro-10-aza-tricyclo[6.3.1.0$^{2,7}$]dodeca-2(7),3,5-triene;
5-fluoro-10-aza-tricyclo[6.3.1.0$^{2,7}$]dodeca-2(7),3,5-triene-4-carbonitrile;
4-ethynyl-5-fluoro-10-aza-tricyclo[6.3.1.0$^{2,7}$]dodeca-2(7),3,5-triene;
5-ethynyl-10-aza-tricyclo[6.3.1.0$^{2,7}$]dodeca-2(7),3,5-triene-4-carbonitrile;
6-methyl-5-thia-5-dioxa-6,13-diazatetracyclo[9.3.1.0$^{2,10}$.0$^{4,8}$]pentadeca-2(10),3,8-triene;
10-aza-tricyclo[6.3.1.0$^{2,7}$]dodeca-2(7),3,5-triene;
4-fluoro-10-aza-tricyclo[6.3.1.0$^{2,7}$]dodeca-2(7),3,5-triene;
4-methyl-10-aza-tricyclo[6.3.1.0$^{2,7}$]dodeca-2(7),3,5-triene;
4-trifluoromethyl-10-aza-tricyclo[6.3.10$^{2,7}$]dodeca-2(7),3,5-triene;
4-nitro-10-azatricyclo[6.3.1.0$^{2,7}$]dodeca-2(7),3,5-triene;
7-methyl-5,7,13-triazatetracyclo[9.3.1.0$^{2,10}$.0$^{4,8}$]pentadeca-2(10),3,5,8-tetraene;
6-methyl-5,7,13-triazatetracyclo[9.3.1.0$^{2,10}$.0$^{4,8}$]pentadeca-2(10),3,5,8-tetraene;
6,7-dimethyl-5,7,13-triazatetracyclo[9.3.1.0$^{2,10}$.0$^{4,8}$]pentadeca-2(10),3,5,8-tetraene;
6-methyl-7-phenyl-5,7,13-triazatetracyclo[9.3.10$^{2,10}$.0$^{4,8}$]pentadeca-2(10),3,5,8-tetraene;
6,7-dimethyl-5,8,14-triazatetracyclo[10.3.1.0$^{2,11}$.0$^{4,9}$]hexadeca-2(11),3,5,7,9-pentaene;
5,8,14-triazatetracyclo[10.3.1.0$^{2,11}$.0$^{4,9}$]hexadeca-2(11),3,5,7,9-pentaene;
14-methyl-5,8,14-triazatetracyclo[10.3.1.0$^{2,11}$.0$^{4,9}$]hexadeca-2(11),3,5,7,9-pentaene;
5-oxa-7,13-diazatetracyclo[9.3.1.0$^{2,10}$.0$^{4,8}$]pentadeca-2(10),3,6,8-tetraene;
6-methyl-5-oxa-7,13-diazatetracyclo[9.3.1.0$^{2,10}$.0$^{4,8}$]pentadeca-2(10),3,6,8-tetraene;
4-chloro-10-azatricyclo[6.3.1.0$^{2,7}$]dodeca-2(7),3,5-triene;
10-azatricyclo[6.3.1.0$^{2,7}$]dodeca-2(7),3,5-trien-4-yl cyanide;
1-(10-azatricyclo[6.3.1.0$^{2,7}$]dodeca-2(7),3,5-trien-4-yl)-1-ethanone;
10-azatricyclo[6.3.1.0$^{2,7}$]dodeca-2(7),3,5-trien-4-ol;
7-methyl-5-oxa-6,13-diazatetracyclo[9.3.1.0$^{2,10}$.0$^{4,8}$]pentadeca-2,4(8),6,9-tetraene;
4,5-dichloro-10-azatricyclo[6.3.1.0$^{2,7}$]dodeca-2(7),3,5-triene;
11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;
1-[11-azatricyclo[7.3.1.0$^{2,7}$]trideca-2(7),3,5-trien-5-yl]-1-propanone;
4-fluoro-11-azatricyclo[7.3.1.0$^{2,7}$]trideca-2(7),3,5-triene-5-carbonitrile;
5-fluoro-11-azatricyclo[7.3.1.0$^{2,7}$]trideca-2(7),3,5-triene-4-carbonitrile;
6-methyl-7-thia-5,14-diazatetracyclo[10.3.1.0$^{2,10}$.0$^{4,8}$]hexadeca-2(10),3,5,8-tetraene;
6-methyl-5,7,14-triazatetracyclo[10.3.1.0$^{2,10}$.0$^{4,8}$]hexadeca-2(10),3,5,8-tetraene;
6,7-dimethyl-5,7,14-triazatetracyclo[10.3.1.0$^{2,10}$.0$^{4,8}$]hexadeca-2(10),3,5,8-tetraene;
5,7,14-triazatetracyclo[10.3.1.0$^{2,10}$.0$^{4,8}$]hexadeca-2(10),3,5,8-tetraene;
5,6-dimethyl-5,7,14-triazatetracyclo[10.3.1.0$^{2,10}$.0$^{4,8}$]hexadeca-2(10),3,6,8-tetraene;
5-methyl-5,7,14-triazatetracyclo[10.3.1.0$^{2,10}$.0$^{4,8}$]hexadeca-2(10),3,6,8-tetraene;
6-(trifluoromethyl)-7-thia-5,14-diazatetracyclo[10.3.1.0$^{2,10}$.0$^{4,8}$]hexadeca-2(10),3,5,8-tetraene;
5,8,15-triazatetracyclo[11.3.1.0$^{2,11}$.0$^{4,9}$]heptadeca-2(11),3,5,7,9-pentaene;
7-methyl-5,8,15-triazatetracyclo[11.3.1.0$^{2,11}$.0$^{4,9}$]heptadeca-2(11),3,5,7,9-pentaene;
6-methyl-5,8,15-triazatetracyclo[11.3.1.0$^{2,11}$.0$^{4,9}$]heptadeca-2(11),3,5,7,9-pentaene;
6,7-dimethyl-5,8,15-triazatetracyclo[11.3.1.0$^{2,11}$.0$^{4,9}$]heptadeca-2(11),3,5,7,9-pentaene;
7-oxa-5,14-diazatetracyclo[10.3.1.0$^{2,10}$.0$^{4,8}$]hexadeca-2(10),3,5,8-tetraene;
6-methyl-7-oxa-5,14-diazatetracyclo[10.3.1.0$^{2,10}$.0$^{4,8}$]hexadeca-2(10),3,5,8-tetraene;
5-methyl-7-oxa-6,14-diazatetracyclo[10.3.1.0$^{2,10}$.0$^{4,8}$]hexadeca-2(10),3,5,8-tetraene;
6-methyl-5-oxa-7,14-diazatetracyclo[10.3.1.0$^{2,10}$.0$^{4,8}$]hexadeca-2(10),3,6,8-tetraene;
7-methyl-5-oxa-6,14-diazatetracyclo[10.3.1.0.$^{2,10}$.0$^{4,8}$]hexadeca-2(10),3,6,8-tetraene;
4,5-difluoro-11-azatricyclo[7.3.1.0$^{2,7}$]trideca-2(7),3,5-triene;
4-chloro-5-fluoro-11-azatricyclo[7.3.1.0$^{2,7}$]trideca-2(7),3,5-triene;
5-chloro-4-fluoro-11-azatricyclo[7.3.1.0$^{2,7}$]trideca-2(7),3,5-triene;
4-(1-ethynyl)-5-fluoro-11-azatricyclo[7.3.1.0$^{2,7}$]trideca-2(7),3,5-triene;
5-(1-ethynyl)-4-fluoro-11-azatricyclo[7.3.1.0$^{2,7}$]trideca-2(7),3,5-triene;
5,6-difluoro-11-aza-tricyclo[7.3.1.0$^{2,7}$]trideca-2,4,6-triene;
6-trifluoromethyl-11-aza-tricyclo[7.3.1.0$^{2,7}$]trideca-2,4,6-triene;
6-methoxy-11-aza-tricyclo[7.3.1.0$^{2,7}$]trideca-2(7),3,5-triene;
11-aza-tricyclo[7.3.1.0$^{2,7}$]trideca-2(7),3,5-trien-6-ol;
6-fluoro-11-aza-tricyclo[7.3.1.0$^{2,7}$]trideca-2(7),3,5-triene;
11-aza-tricyclo[7.3.1.0$^{2,7}$]trideca-2(7),3,5-trien-5-ol;
4-nitro-11-aza-tricyclo[7.3.1.0$^{2,7}$]trideca-2(7),3,5-triene;
5-nitro-11-aza-tricyclo[7.3.1.0$^{2,7}$]trideca-2(7),3,5-triene;
5-fluoro-11-aza-tricyclo[7.3.1.0$^{2,7}$]trideca-2(7),3,5-triene;
6-hydroxy-5-methoxy-11-aza-tricyclo[7.3.1.0$^{2,7}$]trideca-2(7),3,5-triene; and their pharmaceutically acceptable salts and their optical isomers.

**4.** The pharmaceutical composition according to Claim 3 wherein said nicotine receptor partial agonist is selected from:

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
6-methyl-5-thia-5-dioxa-6,13-diazatetracyclo[9.3.1.0$^{2,10}$.0$^{4,8}$]pentadeca-2(10),3,8-triene;
4-fluoro-10-aza-tricyclo[6.3.1.0$^{2,7}$]dodeca-2(7),3,5-triene;
4-trifluoromethyl-10-aza-tricyclo[6.3.1.0$^{2,7}$]dodeca-2(7)3,5-triene;
4-nitro-10-azatricyclo[6.3.1.0$^{2,7}$]dodeca-2(7),3,5-triene;
6-methyl-5,7,13-triazatetracyclo[9.3.1.0$^{2,10}$.0$^{4,8}$]pentadeca-2(10),3,5,8-tetraene;
6,7-dimethyl-5,8,14-triazatetracyclo[10.3.1.0$^{2,11}$.0$^{4,9}$]hexadeca-2(11),3,5,7,9-pentaene;
5,8,14-triazatetracyclo[10.3.1.0$^{2,11}$0.$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;
6-methyl-5-oxa-7,13-diazatetracyclo[9.3.1.0$^{2,10}$.0$^{4,8}$]pentadeca-2(10),3,6,8-tetraene;
10-azatricyclo[6.3.1.0$^{2,7}$]dodeca-2(7),3,5-trien-4-yl cyanide;
1-(10-azatricyclo[6.3.1.0$^{2,7}$]dodeca-2(7),3,5-trien-4-yl)-1-ethanone;
11-azatricyclo[7.3.1.0$^{2,7}$]trideca-2(7),3,5-triene-5-carbonitrile;
1-[11-azatricyclo[7.3.1.0$^{2,7}$]trideca-2(7),3,5-trien-5-yl]-1-ethanone;
1-[11-azatricyclo[7.3.1.0$^{2,7}$]trideca-2(7),3,5-trien-5-yl]-1-propanone;
4-fluoro-11-azatricyclo[7.3.1.0$^{2,7}$]trideca-2(7),3,5-triene-5-carbonitrile;
5-fluoro-11-azatricyclo[7.3.1.0$^{2,7}$]trideca-2(7),3,5-triene-4-carbonitrile;
6-methyl-7-thia-5,14-diazatetracyclo[10.3.1.0$^{2,10}$.0$^{4,8}$]hexadeca-2(10),3,5,8-tetraene;
6-methyl-5,7,14-triazatetracyclo[10.3.1.0$^{2,10}$.0$^{4,8}$]hexadeca-2(10),3,5,8-tetraene;
6,7-dimethyl-5,7,14-triazatetracyclo[10.3.1.0$^{2,10}$.0$^{4,8}$]hexadeca-2(10),3,5,8-tetraene;
6-methyl-7-oxa-5,14-diazatetracyclo[10.3.1.0$^{2,10}$.0$^{4,8}$]hexadeca-2(10),3,5,8-tetraene;
6-methyl-5-oxa-7,14-diazatetracyclo[10.3.1.0$^{2,10}$.0$^{4,8}$]hexadeca-2(10),3,6,8-tetraene;
5,6-difluoro-11-aza-tricyclo[7.3.1.0$^{2,7}$]trideca-2,4,6-triene;
6-trifluoromethyl-11-aza-tricyclo[7.3.1.0$^{2,7}$]trideca-2,4,6-triene;
6-methoxy-11-aza-tricyclo[7.3.1.0$^{2,7}$]trideca-2(7),3,5-triene;
6-fluoro-11-aza-tricyclo[7.3.1.0$^{2,7}$]trideca-2(7),3,5-triene;
11-aza-tricyclo[7.3.1.0$^{2,7}$]trideca-2(7),3,5-trien-5-ol, and their pharmaceutically acceptable salts and their optical isomers thereof.

**5.** The pharmaceutical composition according to claim 1, wherein the $D_2$ receptor antagonists are selected from pergolide, bromocriptine, ropinirole, pramipexole, pemoline, cabergoline, amphetamine, methylphenidate, dextroamphetamine and their pharmaceutically acceptable salts.

**6.** The pharmaceutical composition according to claim 1, wherein the $5HT_1A$ receptor agonists are selected from buspirone, gepirone, ipsapirone, flesinoxan, sunepitron and bis- azabicycle compounds of formula I as defined in the specification and their pharmaceutically acceptable salts.

**7.** The pharmaceutical composition according to claim 2, wherein the $\alpha_2$ adreneric receptor antagonists agent are selected from yohimbine, idazoxan and clonidine and their pharmaceutically acceptable salts.

**8.** The pharmaceutically composition according to claim 1, wherein the $\alpha_1$ adrenergic receptor agonists are selected from modafinil and its pharmaceutically acceptable salt.

**9.** The pharmaceutical composition according to claim 1, wherein the norepinephrine re-uptake inhibitors are selected from tomoxetine, buproprian, and venlafaxtine and their pharmaceutically acceptable salts.

**10.** The pharmaceutical composition according to claim 1, wherein the cholinesterase inhibitors are selected from donepezil, tacrine, rivastigmine, physostigmine, galanthamine, metrifonate, neostigmine, and icopezil and their pharmaceutically acceptable salts.

**11.** A method of treating ADHD comprising administering to a mammal respectively

(a) a nicotine receptor partial agonist or a pharmaceutically acceptable salt thereof;
(b) an $\alpha_2$ adrenergic receptor ligand, an $\alpha_1$ adrenergic receptor ligand, a $D_2$ receptor agonist, a NEURI, a $5HT_{1A}$ receptor agonist, and/or a cholinesterase inhibitor or a pharmaceutically acceptable salt thereof; and
(c) a pharmaceutically acceptable carrier;

wherein the active agents "a" and "b" above are present in amounts that render the composition effective in treating Attention Deficit Hyperactivity Disorder (ADHD).

**12.** The method according to claim 11 wherein the $\alpha_2$ adrenergic receptor ligand that is employed is a presynaptic $\alpha_2$ adrenergic antagonist or a postynaptic $\alpha_2$ adrenergic receptor agoinst.

**13.** The method according to claim 11 wherein the nicotine partial agonist is selected from:

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1,5]diazocin-8-one;
9-(2-propyl)-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(26-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one;
9-(3,5-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(25-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.0$^{2,10}$.0$^{4,8}$]pentadeca-2(10),3,8-triene;
5-oxo-6,13-diazatetracyclo[9.3.1.0$^{2,10}$.0$^{4,8}$]pentadeca-2(10),3,8-triene;
6-oxo-5,7,13-triazatetracyclo[9.3,1.0$^{2,10}$.0$^{4,8}$]pentadeca-2(10),3,8-triene;
4,5-difluoro-10-aza-tricyclo[6.3.1.0$^{2,7}$]dodeca-2(7)3,5-triene;
5-fluoro-10-aza-tricyclo[6.3.10$^{2,7}$]dodeca-2(7),3,5-triene-4-carbonitrile;
4-ethynyl-5-fluoro-10-aza-tricyclo[6.3.1.0$^{2,7}$]dodeca-2(7),3,5-triene;
5-ethynyl-10-aza-tricyclo[6.3.1.0$^{2,7}$]dodeca-2(7),3,5-triene-4-carbonitrile;
6-methyl-5-thia-5-dioxa-6,13-diazatetracyclo[9.3.1.0$^{2,10}$.0$^{4,8}$]pentadeca-2(10),3,8-triene;
10-aza-tricyclo[6.3.1.0$^{2,7}$]dodeca-2(7),3,5-triene;
4-fluoro-10-aza-tricyclo[6.3.1.0$^{2,7}$]dodeca-2(7),3,5-triene;
4-methyl-10-aza-tricyclo[6.3.1.0$^{2,7}$]dodeca-2(7),3,5-triene;
4-trifluoromethyl-10-aza-tricyclo[6.3.1.0$^{2,7}$]dodeca-2(7),3,5-triene;
4-nitro-10-azatricyclo[6.3.1.0$^{2,7}$]dodeca-2(7),3,5-triene;
7-methyl-5,7,13-triazatetracyclo[9.3.1.0$^{2,10}$.0$^{4,8}$]pentadeca-2(10),3,5,8-tetraene;
6-methyl-5,7,13-triazatetracyclo[9.3.1.0$^{2,10}$.0$^{4,8}$]pentadeca-2(10),3,5,8-tetraene;
6,7-dimethyl-5,7,13-triazatetracyclo[9.3.1.0$^{2,10}$.0$^{4,8}$]pentadeca-2(10),3,5,8-tetraene;
6-methyl-7-phenyl-5,7,13-triazatetracyclo[9.3.1.0$^{2,10}$.0$^{4,8}$]pentadeca-2(10),3,5,8-tetraene;
6,7-dimethyl-5,8,14-triazatetracyclo[10.3.1.0$^{2,11}$.0$^{4,9}$]hexadeca-2(11),3,5,7,9-pentaene;
5,8,14-triazatetracyclo[10.3.1.0$^{2,11}$.0$^{4,9}$]hexadeca-2(11),3,5,7,9-pentaene;
14-methyl-5,8,14-triazatetracyclo[10.3.1.0$^{2,11}$.0$^{4,9}$]hexadeca-2(11),3,5,7,9-pentaene;
5-oxa-7,13-diazatetracyclo[9.3.1.0$^{2,10}$.0$^{4,8}$]pentadeca-2(10),3,6,8-tetraene;
6-methyl-5-oxa-7,13-diazatetracyclo[9.3.1.0$^{2,10}$.0$^{4,8}$]pentadeca-2(10),3,6,8-tetraene;
4-chloro-10-azatricyclo[6.3.1.0$^{2,7}$]dodeca-2(7),3,5-triene;
10-azatricyclo[6.3.1.0$^{2,7}$]dodeca-2(7),3,5-trien-4-yl cyanide;
1-(10-azatricyclo[6.3.1.0$^{2,7}$]dodeca-2(7),3,5-trien-4-yl)-1-ethanone;
10-azatricyclo[6.3.1.0$^{2,7}$]dodeca-2(7),3,5-trien-4-ol;
7-methyl-5-oxa-6,13-diazatetracyclo[9.3.1.0$^{2,10}$.0$^{4,8}$]pentadeca-2,4(8)6,9-tetraene;
4,5-dichloro-10-azatricyclo[6.3.1.0$^{2,7}$]dodeca-2(7),3,5-triene;
11-azatricyclo[7.3.1.0$^{2,7}$]trideca-2(7),3,5-triene-5-carbonitrile;
1-[11-azatricyclo[7.3.1.0$^{2,7}$]trideca-2(7),3,5-trien-5-yl]-1-ethanone;
1-[11-azatricyclo[7.3.1.0$^{2,7}$]trideca-2(7),3,5-trien-5-yl]-1-propanone;
4-fluoro-11-azatricyclo[7.3.1.0$^{2,7}$]trideca-2(7),3,5-triene-5-carbonitrile;
5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;
6-methyl-5,7,14-triazatetracyclo[10.3.1.0$^{2,10}$.0$^{4,8}$]hexadeca-2(10),3,5,8-tetraene;
6,7-dimethyl-5,7,14-triazatetracyclo[10.3.1.0$^{2,10}$.0$^{4,8}$]hexadeca-2(10),3,5,8-tetraene;
5,7,14-triazatetracyclo[10.3.1.0$^{2,10}$.0$^{4,8}$]hexadeca-2(10),3,5,8-tetraene;
5,6-dimethyl-5,7,14-triazatetracyclo[10.3.1.0$^{2,10}$.0$^{4,8}$]hexadeca-2(10),3,6,8-tetraene;
5-methyl-5,7,14-triazatetracyclo[10.3.1.0$^{2,10}$.0$^{4,8}$]hexadeca-2(10),3,6,8-tetraene;
6-(trifluoromethyl)-7-thia-5,14-diazatetracyclo[10.3.1.0$^{2,10}$.0$^{4,8}$]hexadeca-2(10),3,5,8-tetraene;
5,8,15-triazatetracyclo[11.3.1.0$^{2,11}$.0$^{4,9}$]heptadeca-2(11),3,5,7,9-pentaene;
7-methyl-5,8,15-triazatetracyclo[11.3.1.0$^{2,11}$.0$^{4,9}$]heptadeca-2(11),3,5,7,9-pentaene;
6-methyl-5,8,15-triazatetracyclo[11.3.1.0$^{2,11}$.0$^{4,9}$]heptadeca-2(11 ),3,5,7,9-pentaene;
6,7-dimethyl-5,8,15-triazatetracyclo[11.3.1.0$^{2,11}$.0$^{4,9}$]heptadeca-2(11),3,5,7,9-pentaene;
7-oxa-5,14-diazatetracyclo[10.3.1.0$^{2,10}$.0$^{4,8}$]hexadeca-2(10),3,5,8-tetraene;
6-methyl-7-oxa-5,14-diazatetracyclo[10.3.10$^{2,10}$.0$^{4,8}$]hexadeca-2(10),3,5,8-tetraene;
5-methyl-7-oxa-6,14-diazatetracyclo[10.3.1.0$^{2,10}$.0$^{4,8}$]hexadeca-2(10),3,5,8-tetraene;
6-methyl-5-oxa-7,14-diazatetracyclo[10.3.1.0$^{2,10}$.0$^{4,8}$]hexadeca-2(10),3,6,8-tetraene;
7-methyl-5-oxa-6,14-diazatetracyclo[10.3.1.$^{2,10}$.0$^{4,8}$]hexadeca-2(10),3,6,8-tetraene;
4,5-difluoro-11-azatricyclo[7.3.1.0$^{2,7}$]trideca-2(7),3,5-triene;
4-chloro-5-fluoro-11-azatricyclo[7.3.1.0$^{2,7}$]trideca-2(7),3,5-triene;
5-chloro-4-fluoro-11-azatricyclo[7.3.1.0$^{2,7}$]trideca-2(7),3,5-triene;
4-(1-ethynyl)-5-fluoro-11-azatricyclo[7.3.1.0$^{2,7}$]trideca-2(7),3,5-triene;
5-(1-ethynyl)-4-fluoro-11-azatricyclo[7.3.1.0$^{2,7}$]trideca-2(7),3,5-triene;
5,6-difluoro-11-aza-tricyclo[7.3.1.0$^{2,7}$]trideca-2,4,6-triene;
6-trifluoromethyl-11-aza-tricyclo[7.3.1.0$^{2,7}$]trideca-2,4,6-triene;
6-methoxy-11-aza-tricyclo[7.3.10$^{2,7}$]trideca-2(7),3,5-triene;
11-aza-tricyclo[7.3.1.0$^{2,7}$]trideca-2(7),3,5-trien-6-ol;
6-fluoro-11-aza-tricyclo[7.3.1.0$^{2,7}$]trideca-2(7),3,5-triene;
11-aza-tricyclo[7.3.1.0$^{2,7}$]trideca-2(7),3,5-trien-5-ol;
4-nitro-11-aza-tricyclo[7.3.1.0$^{2,7}$]trideca-2(7),3,5-triene;
5-nitro-11-aza-tricyclo[7.3.1.0$^{2,7}$]trideca-2(7),3,5-triene;
5-fluoro-11-aza-tricyclo[7.3.1.0$^{2,7}$]trideca-2(7),3,5-triene;
6-hydroxy-5-methoxy-11-aza-tricyclo[7.3.1.0$^{2,7}$]trideca-2(7),3,5-triene and a pharmaceutically acceptable salt and an optical isomer thereof.

**14.** The method according to claim 13, wherein the nicotine partial agonist is selected from

9-bromo-1,2,3,4,5,6-hexahydro-1 ,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
6-methyl-5-thia-5-dioxa-6,13-diazatetracyclo[9.3.1.0$^{2,10}$.0$^{4,8}$]pentadeca-2(10),3,8-triene;
4-fluoro-10-aza-tricyclo[6.3.1.0$^{2,7}$]dodeca-2(7),3,5-triene;
4-trifluoromethyl-10-aza-tricyclo[6.3.1.0$^{2,7}$]dodeca-2(7),3,5-triene;
4-nitro-10-azatricyclo[6.3.1.0$^{2,7}$]dodeca-2(7),3,5-triene;
6-methyl-5,7,13-triazatetracyclo[9.3.1.0$^{2,10}$.0$^{4,8}$]pentadeca-2(10),3,5,8-tetraene;
6,7-dimethyl-5,8,14-triazatetracyclo[10.3.1.0$^{2,11}$.0$^{4,9}$]hexadeca-2(11 ),3,5,7,9-pentaene;
5,8,14-triazatetracyclo[10.3.1.0$^{2,11}$.0$^{4,9}$]hexadeca-2(11),3,5,7,9-pentaene;
5-oxa-7,13-diazatetracyclo[9.3.1.0$^{2,10}$.0$^{4,8}$]pentadeca-2(10),3,6,8-tetraene;
6-methyl-5-oxa-7,13-diazatetracyclo[9.3.1.0$^{2,10}$.0$^{4,8}$]pentadeca-2(10),3,6,8-tetraene;
10-azatricyclo[6.3.1.0$^{2,7}$]dodeca-2(7),3,5-trien-4-yl cyanide;
1-(10-azatricyclo[6.3.1.0$^{2,7}$]dodeca-2(7),3,5-trien-4-yl)-1-ethanone;
11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;
1-[11-azatricyclo[7.3.1.0$^{2,7}$]trideca-2(7),3,5-trien-5-yl]-1-propanone;
4-fluoro-11-azatricyclo[7.3.1.0$^{2,7}$]trideca-2(7),3,5-triene-5-carbonitrile;
5-fluoro-11-azatricyclo[7.3.1.0$^{2,7}$]trideca-2(7),3,5-triene-4-carbonitrile;
6-methyl-7-thia-5,14-diazatetracyclo[10.3.1.0$^{2,10}$.0$^{4,8}$]hexadeca-2(10),3,5,8-tetraene;
6-methyl-5,7,14-triazatetracyclo[10.3.1.0$^{2,10}$.0$^{4,8}$]hexadeca-2(10),3,5,8-tetraene;
6,7-dimethyl-5,7,14-triazatetracyclo[10.3.1.0$^{2,10}$.0$^{4,8}$]hexadeca-2(10),3,5,8-tetraene;
6-methyl-7-oxa-5,14-diazatetracyclo[10.3.10$^{2,10}$.0$^{4,8}$]hexadeca-2(10),3,5,8-tetraene;
6-methyl-5-oxa-7,14-diazatetracyclo[10.3.1.0$^{2,10}$.0$^{4,8}$]hexadeca-2(10),3,6,8-tetraene;
5,6-difluoro-11-aza-tricyclo[7.3.1.0$^{2,7}$]trideca-2,4,6-triene;
6-trifluoromethyl-11-aza-tricyclo[7.3.1.0$^{2,7}$]trideca-2,4,6-triene;
6-methoxy-11-aza-tricyclo[7.3.1.0$^{2,7}$]trideca-2(7),3,5-triene;
6-fluoro-11-aza-tricyclo[7.3.1.0$^{2,7}$]trideca-2(7),3,5-triene;
11-aza-tricyclo[7.3.1.0$^{2,7}$]trideca-2(7),3,5-trien-5-ol; and the pharmaceutically acceptable salts and optical isomers thereof.

**15.** The method according to claim 11, wherein the $D_2$ receptor agonist are selected from pergolide, bromocriptine, ropinirole, pramipexole, pemoline, cabergoline, amphetamine and methylphenidate, and dextroamphetaime and their pharmaceutically acceptable salts.

**16.** The method according to claim 11 wherein the $5HT_{1A}$ receptor agonists are selected from buspirone, gepirone, ipsapirone, flesinoxan, sunepitron and bis-azabicycle compounds of formula I defined in the specification and their pharmaceutically acceptable salts.

**17.** The method according to claim 12, wherein the $\alpha_2$ adreneric receptor antagonists are selected from yohimbine idazoxan and clonidine and their pharmaceutically acceptable salts.

**18.** The method according to claim 11 wherein the $\alpha_1$ andrenergic receptor agonists are selected from modafinil and their pharmaceutically acceptable salts.

**19.** The method according to claim 11 wherein norepinephrine re-uptake inhibitors are selected from tomoxetine, bupropion, and venlafaxine and their pharmaceutically acceptable salts.

**20.** The method according to claim 11 wherein the cholinesterase inhibitors are selected from donepezil, tacrine, rivastigmine, physostigmine, galanthamine, metrifonate, neostigmine, and copezil and their pharmaceutically acceptable salts.